# FASCICULE DE BREVET EUROPEEN

(11) **EP 0 719 254 B1**
(45) Date de publication et mention de la délivrance du brevet: **30.05.2001**
(21) Numéro de dépôt: 94926984.9
(22) Date de dépôt: 14.09.1994
(51) Int. Cl.: C07D 209/60, C09B 23/08

(54) **PROCEDE DE PREPARATION DE BENZ E]INDOLES SUBSTITUES DE PURETE ELEVEE ET LEURS SELS ALCALINS**
VERFAHREN ZUR HERSTELLUNG VON SUBSTITUIERTEN BENZ [E] INDOLEN MIT HOHER REINHEIT UND IHREN ALKALISALZEN
METHOD FOR THE PREPARATION OF HIGH PURITY SUBSTITUTED BENZ E]INDOLES AND THE ALKALINE SALTS THEREOF

(30) Priorité: 17.09.1993 FR 9311121
(43) Date de publication de la demande: 03.07.1996
(73) Titulaire: Societe d'Etudes et de Recherches Biologiques, 75020 Paris (FR)
(72) Inventeur: HUYNH, Anh, Hoa, F-75010 Paris (FR); TRAN, Joanne, F-95100 Argenteuil (FR); SCHERNINSKI, François, F-75011 Paris (FR); GUETTE, Jean-Paul, F-75006 Paris (FR)
(74) Mandataire: Burtin, Jean-François
(86) Numéro de dépôt international: FR9401077
(87) Numéro de publication internationale: WO9507888

(56) Documents cités:
- DE-A- 2 046 141
- US-A- 2 895 955
- CHEMICAL ABSTRACTS, vol. 114, no. 18, 6 Mai 1991, Columbus, Ohio, US; abstract no. 175012e, ARAKAWA, DEIICHI ET AL. 'Heat-mode optical recording medium containing cyanine dyes.' & JP,A,02 164 586 (SONY CORP.)
- CHEMICAL ABSTRACTS, vol. 116, no. 16, 20 Avril 1992, Columbus, Ohio, US; abstract no. 162619z, ATA, AKIFUMI ET AL. 'Optical recording media containing benzoindoleine pentamethinecyanine dyes.' & JP,A,03 272 890 (SONY CORP.)

## Description

L'invention a pour objet un procédé de préparation de benz[e]-indoles substitués de pureté élevée répondant à la formule générale 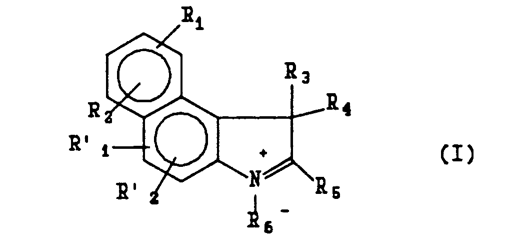 dans laquelle
- R₁, R₂,R'₁, R'₂, R₃, R₄ et R₅, qui sont identiques ou différents les uns des autres, représentent des atomes d'hydrogène, des groupes alkyle en C₁ à C₁₂, des groupes sulfoalkyle, cycloalkyle, alkoxyle en C₁ à C₄, des groupes aryle, aroxyle, ou des atomes d'halogène, et
- R₆ représente un groupe sulfoalkyle, halogénoalkyle ou hydroxycarbonylalkyle en C₁ à C₇, de préférence en C₁ à C₄,
R₅ pouvant également représenter un groupement de formule 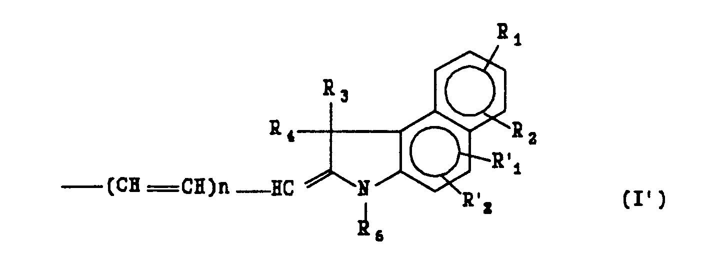 dans laquelle n représente un nombre entier de 1 à 7.

Elle vise également la préparation des sels alcalins des benz[e]indoles substitués de formule (I).

Elle a plus particulièrement pour objet l'application du susdit procédé à la préparation du Vert d'Indocyanine ou sel interne, sel de sodium de l'hydroxyde de 2-[7[1,3-dihydro-1,1-diméthyl-3-(4-sulfobutyl)-2H-benz [e]-indol-2-ylidène] -1,3,5-heptatriényl]-1,1-diméthyl-3-(4-sulfobutyl)-lH-benz[e]indolium représenté par la formule:

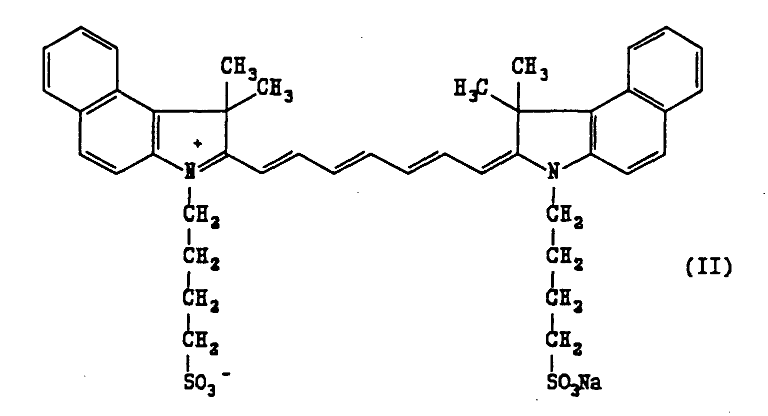

Les benz[e]indoles substitués de formule (I) sont le plus souvent des colorants utilisés notamment dans l'industrie.

Ainsi, le Vert d'Indocyanine est un colorant essentiellement destiné à l'industrie photographique.

Il a également été proposé d'avoir recours au Vert d'Indocyanine en tant que colorant dans l'industrie pharmaceutique, notamment comme auxiliaire de diagnostic, en particulier pour la détermination du volume sanguin, du débit cardiaque et pour le contrôle de la fonction hépatique.

Or, si le Vert d'Indocyanine n'a jamais pu s'imposer véritablement en tant que colorant dans le domaine de l'industrie pharmaceutique, c'est en raison du fait que les procédés connus pour la préparation des benz[e]indoles de formule (I) en général et du Vert d'Indocyanine en particulier conduisent à des produits qu'il est impossible d'amener d'un point de vue pratique à un degré de pureté suffisant pour éviter tous phénomènes allergisants, voire toxiques.

En effet, les produits du genre en question qui se trouvent déjà dans le commerce présentent, de par leur procédé de préparation, notamment un taux résiduel d'ions iodure qu'il est impossible d'amener dans des conditions économiques acceptables à un niveau suffisamment bas pour éviter tout phénomène toxique.

Il a bien été proposé d'éviter l'utilisation de l'iode dans la préparation des produits du genre en question mais il se trouve que les solutions envisagées mettent en oeuvre des solvants toxiques, notamment le méthanol.

De plus, les procédés connus mettent en oeuvre des amines cancérigènes, notamment la β-naphtylamine.

L'invention a donc pour but, surtout, de fournir un procédé de préparation des benz[e]indoles substitués de formule (I) et en particulier du Vert d'Indocyanine qui ne présente plus les inconvénients des procédés de l'art antérieur et qui conduise, par voie de conséquence, à des produits de pureté élevée, substantiellement exempts d'ions iodure, de traces d'amines cancérigènes et de solvants toxiques.

Et la Société Demanderesse a eu le mérite d'avoir mis au point un procédé permettant d'atteindre ce but et caractérisé par le fait que successivement
- on prépare une arylhydrazine de formule: 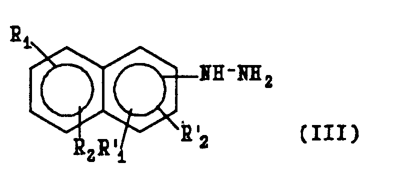 par réaction de l'hydrazine avec l'arylhydroxyle correspondant, évitant ainsi tout recours à des amines cancérigènes,
- on effectue une synthèse indolique de Fischer entre l'arylhydrazine de formule (III) et une cétone de formule: 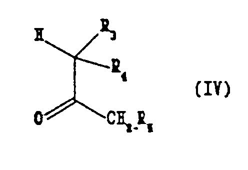 ce qui fournit un benz[e]indole de formule: 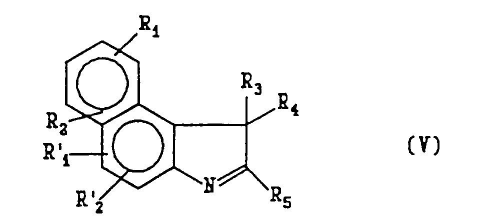
- on forme le benz[e]indole de formule (I) par réaction du benz[e]indole de formule (V) avec le radical R₆ qui représente un groupe sulfoalkyle, halogénoalkyle ou hydroxycarbonylalkyle en C₁ à C₇, de préférence en C₁ à C₄, les significations de R₁, R₂, R'₁, R'₂, R₃, R₄ et R₅ étant celles données en rapport avec la formule (I), à la différence près que R₅ ne peut représenter un groupement de formule (I'),
- on transforme, le cas échéant, le benz[e]indole de formule (I) ainsi préparé en sel alcalin soluble par réaction avec un alcoolate alcalin ou un sel alcalin d'un acide organique, en particulier avec l'acétate de sodium, le benz[e]indole de formule (I) ou, s'il a été préparé, le sel alcalin correspondant étant débarrassé de ses impuretés résiduelles, par extraction à l'aide d'un solvant apolaire dont le point d'ébullition est proche de celui de l'acétone, ce solvant pouvant être choisi dans le groupe comprenant notamment le pentane, l'hexane, l'heptane, la cyclohexane et l'éther de pétrole.

Selon un mode de réalisation particulier du susdit procédé, on prépare le benz[e]indole de formule (I) dans laquelle R₅ représente le susdit groupement de formule (I'), en faisant réagir d'abord le dérivé sulfonique du benz[e)indole de formule (I) avec le chlorhydrate de dianilide de l'aldéhyde glutaconique, puis le produit ainsi obtenu avec une deuxième molécule du susdit dérivé sulfonique du benz[e]indole de formule (I).

Selon un autre mode de réalisation avantageux du susdit procédé, appliqué à la synthèse du Vert d'Indocyanine, successivement
- on prépare le benz[e]indole de formule (V) dans laquelle, d'une part, R₁, R₂, R'₁, R'₂ sont des atomes d'hydrogène et, d'autre part, R₃, R₄ et R₅ des radicaux méthyle, par réaction de la 2-naphtylhydrazine avec l'isopropylméthylcétone,
- on fait réagir le benz[e]indole ainsi obtenu avec la 1,4-butane-sultone,
- on condense en milieu acétique le résultat de cette réaction avec le chlorhydrate de dianilide de l'aldéhyde glutaconique et
- on fait réagir en milieu éthanolique le produit ainsi formé avec une deuxième molécule du benz[e]indole de formule (V) comportant sur l'atome d'azote le même radical 1,4-butane-sultone, la conversion en sel hydrosoluble étant effectuée en introduisant directement dans le milieu réactionnel un alcoolate alcalin ou un sel alcalin d'un acide organique, en particulier l'acétate de sodium.

La préparation de l'arylhydrazine est mise en oeuvre en milieu aqueux, à chaud, notamment à une température d'environ 100 à environ 160°C et sous pression, notamment d'environ 50 à 150 bars.

Il est rappelé que le principe de la synthèse indolique de Fischer est de faire réagir en milieu acide une arylhydrazine avec une cétone convenablement choisie (voir par exemple "*The Fischer Indole Synthesis*" par B. Robinson, Chem. Rev., vol. 63 (1963), pages 371-401.

L'invention pourra être encore mieux comprise à l'aide de l'exemple qui suit et qui concerne plus particulièrement la préparation du Vert d'Indocyanine.

### EXEMPLE

### Préparation de la 2-naphtylhydrazine

Dans un autoclave de 500 ml, on introduit 57,6 g (0,4 mol) de 2-naphtol et 200 ml d'hydrazine (4 mol). Le mélange est agité à 85°C sous une pression de 60 bars pendant environ 100 heures. Le milieu réactionnel est ensuite extrait avec du dichlorométhane. La phase organique est lavée avec de la soude à 10%, puis à l'eau et enfin à l'eau salée; elle est séchée sur du sulfate de magnésium. Après filtration et évaporation sous vide, on obtient 50,6 g (80%) de produit dont le point de fusion est de 124°C (alcool/eau).

### Préparation du Vert d'Indocyanine sous forme acide

On fait réagir 50 g (0,32 mol) de 2-naphtylhydrazine avec 48 ml (0,45 mol) d'isopropylméthylcétone en milieu acétique aqueux.

On obtient ainsi 46,8 g (70%) de 1,1,2-triméthyl-benz[e]indole (point de fusion 114°C) auquel on ajoute 33 g (0,24 mole) de 1,4-butane-sultone.

Après lavage à l'acétone, on recueille 68 g (90%) de dérivé sulfonique.

On fait réagir sous agitation les 34 g de ce dérivé avec 31 g (0,11 mol) de chlorhydrate de dianilide de l'aldéhyde glutaconique dans 170 ml d'anhydride acétique, la température étant de 130°C.

On refroidit à la température ambiante et on continue l'agitation pendant 30 minutes.

Il se forme un précipité d'hydroxyde d'anhydro-2-(6-acétanilido-1,3,5-hexatriényl)-3,3-diméthyl-1-(4-sulfobutyl)-4,5-benzopseudoindolium qui est lavé à l'acétone puis séché.

La masse du produit hexatriénique séché ainsi obtenu est de 42,7 g (80%); son point de fusion est de 168-170°C avec décomposition.

On dissout 13,8 g (25,5 mmol) de ce dernier produit dans 80 ml d'éthanol anhydre et on ajoute 8,8 g (25,5 mmol) du dérivé sulfonique obtenu plus haut; après addition de 2,6 g (25,5 mmol) de triéthylamine, on chauffe à reflux pendant 15 minutes; après refroidissement, on obtient un précipité (15,8 g) de Vert d'Indocyanine sous forme acide.

### Préparation du sel sodique du Vert d'Indocyanine

On dissout 42,7 g (78,8 mmol) du susdit produit hexatriénique dans 250 ml d'éthanol anhydre et on ajoute 27,3 g (78,8 mmol) du dérivé sulfonique obtenu plus haut en même temps que 8 g (78,8 mmol) de triéthylamine; après refroidissement, on ajoute directement dans le milieu réactionnel 6,5 g (78,8 mmol) d'acétate de sodium préalablement dissous dans 450 ml d'éthanol anhydre sous agitation pendant 30 minutes.

Le sel formé est essoré, lavé à l'acétone et séché.

On obtient 15,8 g (80%) du produit recherché dont le point de fusion est de 243°C (avec décomposition).

### Purification

Le susdit Vert d'Indocyanine sous forme de sel sodique est purifié par extraction des impuretés sous reflux en utilisant comme solvant l'acétone.

Le produit obtenu, exempt d'ions iodure, contient moins de 0,5% d'impuretés résiduelles; il est adapté à toutes applications dans le domaine pharmaceutique et notamment comme colorant en angiographie infrarouge.

## Revendications

1. Procédé de préparation de benz[e]-indoles substitués, débarrassés de leurs impuretés résiduelles, substantiellement exempts d'ions iodures et de traces d'amines cancérigènes, répondant à la formule générale (I) : 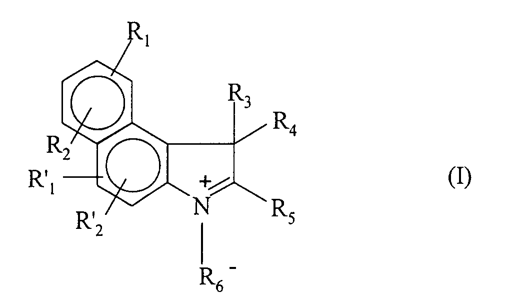 dans laquelle
- R₁, R₂, R'₁, R'₂, R₃ et R₄, qui sont identiques ou différents les uns des autres, représentent des atomes d'hydrogène, des groupes alkyle en C₁ à C₁₂, des groupes sulfoalkyle, cycloalkyle, alkoxyle en C₁ à C₄, des groupes aryle, aroxyle, ou des atomes d'halogène, et
- R₆ représente un groupe sulfoalkyle, en C₁ à C₇, de préférence en C₁ à C₄, et
- R₅ représente un atome d'hydrogène, un groupe alkyle en C₁ à C₁₂, un groupe sulfoalkyle, cycloalkyle ou alkoxyle en C₁-C₄, un groupe aryle ou aroxyle, un atome d'halogène ou un groupement de formule (I') 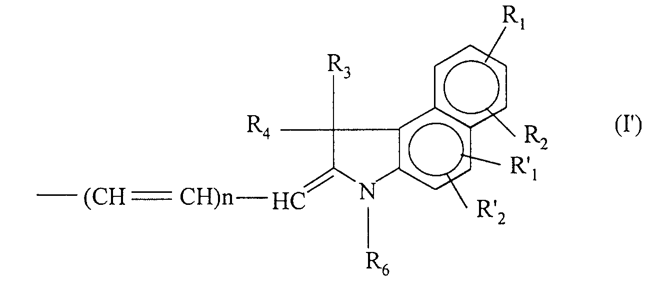 dans laquelle n représente un nombre entier de 1 à 7, à la condition que lorsque R₁, R₂, R'₁ et R'₂ représentent l'hydrogène, et R₃, R₄ le méthyle, R₅ ne représente pas le méthyle, caractérisé par le fait que successivement :
- on prépare une arylhydrazine de formule (III) : 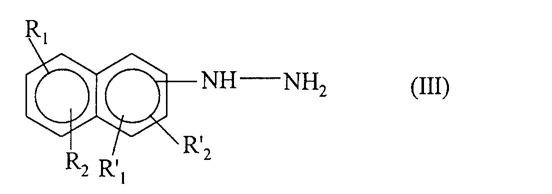 par réaction de l'hydrazine avec l'arylhydroxyle correspondant,
- on effectue une synthèse indolique de Fischer entre l'arylhydrazine de formule (III) et une cétone de formule (IV) : 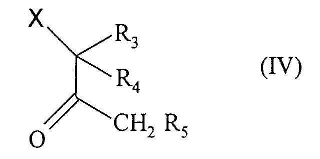 ce qui fournit un benz[e]indole de formule (V) : 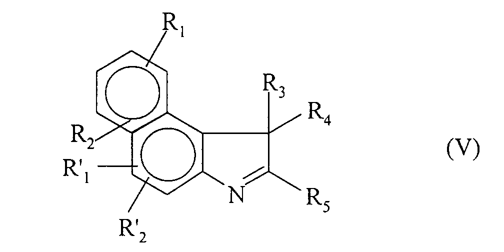
- on forme le dérivé sulfonique du benz[e]indole de formule (I) par réaction du benz[e]indole de formule (V) avec un sulfoalkyle en C₁-C₇, les significations de R₁, R₂, R'₁, R'₂, R₃, R₄, et R₅ étant celles données en rapport avec la formule (I), à la différence près que R₅ ne peut représenter un groupement de formule (I'),
- on transforme le benz[e]indole de formule (I) ainsi préparé en sel alcalin soluble par réaction avec un alcoolate alcalin ou un sel alcalin d'un acide organique, étant entendu que lorsque R₅ représente le groupement de formule (I'), l'on fait réagir d'abord le dérivé sulfonique de benz[e]indole de formule (I) avec le chlorhydrate de dianilide de l'aldéhyde glutaconique, puis le produit ainsi obtenu avec une deuxième molécule du dérivé sulfonique de benz[e]indole de formule (I),
- et on débarrasse le sel alcalin ainsi obtenu de ses impuretés résiduelles, par extraction ou reflux à l'aide d'un solvant apolaire dont le point d'ébullition est proche de celui de l'acétone.

2. Procédé selon la revendication 1, caractérisé par le fait que, pour préparer le sel alcalin du Vert d'Indocyanine, successivement :
- on prépare le benz[e]indole de formule (V) dans laquelle, d'une part R₁, R₂, R'₁ et R'₂ sont des atomes d'hydrogène et, d'autre part, R₃, R₄ et R₅ sont des radicaux méthyle, par réaction de la 2-naphtylhydrazine avec l'isopropylméthylcétone,
- on fait réagir le benz[e]indole ainsi obtenu avec la 1,4-butane-sultone,
- on condense en milieu acétique le résultat de cette réaction avec le chlorhydrate de dianilide de l'aldéhyde glutaconique, et
- on fait réagir en milieu éthanolique le produit ainsi formé avec une deuxième molécule du benz[e]indole de formule (V) comportant sur l'atome d'azote le même radical sulfobutyle, la conversion en sel hydrosoluble étant effectuée en introduisant directement dans le milieu réactionnel un alcoolate alcalin ou un sel alcalin d'un acide organique,
- on débarrasse le produit obtenu par extraction ou reflux à l'aide d'acétone.

3. Procédé de préparation du Vert d'Indocyanine contenant moins de 0.5% d'impuretés résiduelles, substantiellement exempt d'ions iodures et de traces d'amines cancérigènes, répondant à la formule générale (II) : 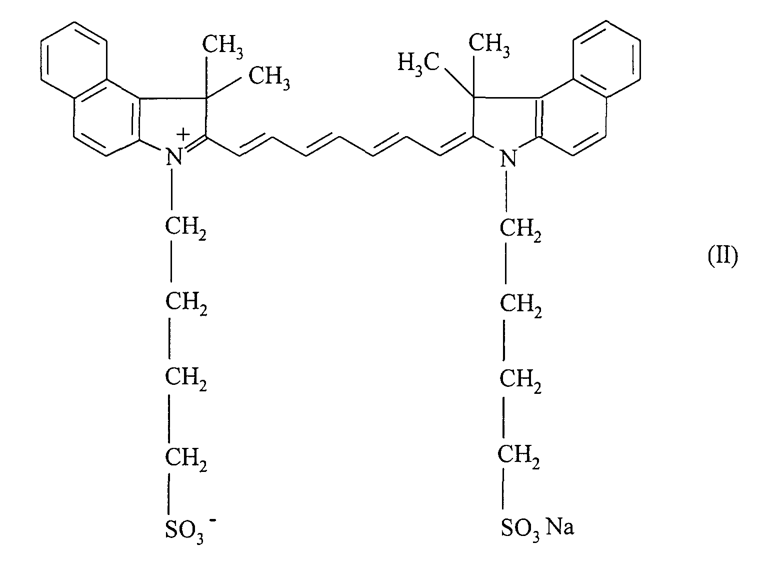 caractérisé par le fait que successivement :
- on prépare une arylhydrazine de formule (III) : 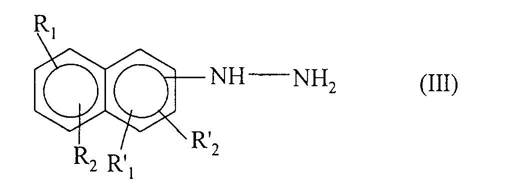 dans laquelle R₁, R₂, R'₁ et R'₂ représentent des atomes d'hydrogène, par réaction de l'hydrazine avec l'arylhydroxyle correspondant,
- on effectue une synthèse indolique de Fischer entre l'arylhydrazine de formule (III) et une cétone de formule (IV) : 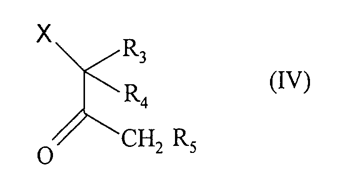 ce qui fournit un benz[e]indole de formule (V) : 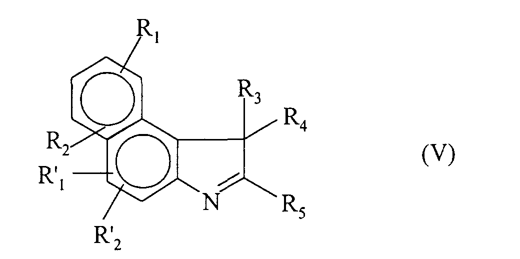 dans laquelle R₃, R₄ et R₅ représentent des groupes méthyle,
- on forme le dérivé sulfonique du benz[e]indole de formule (II) par réaction du benz[e]indole de formule (V) avec un sulfoalkyle en C₁-C₇,
- on fait réagir le dérivé sulfonique du benz[e]indole de formule (II) avec le chlorhydrate de dianilide de l'aldéhyde glutaconique, puis le produit ainsi obtenu avec une deuxième molécule du dérivé sulfonique de benz[e]indole de formule (II),
- on transforme le produit ainsi préparé en sel alcalin soluble par réaction avec un alcoolate alcalin ou un sel alcalin d'un acide organique, et on purifie le sel ainsi obtenu par extraction ou reflux à l'aide d'acétone.

4. Procédé selon la revendication 3, caractérisé par le fait que, pour préparer le sel alcalin du Vert d'Indocyanine, successivement :
- on prépare le ben[z]indole de formule (V) dans laquelle, d'une part R₁, R₂, R'₁, R'₂ sont des atomes d'hydrogène et, d'autre part, R₃, R₄ et R₅ sont des radicaux méthyle, par réaction de la 2-naphtylhydrazine avec l'isopropylméthylcétone,
- on fait réagir le benz[e]indole ainsi obtenu avec la 1,4-butane-sulfone,
- on condense en milieu acétique le produit de cette réaction, avec le chlorhydrate de dianilide de l'aldéhyde glutaconique et
- on fait réagir en milieu éthanolique le produit ainsi formé avec une deuxième molécule de benz[e]indole de formule (V) comportant sur l'atome d'azote le même radical sulfobutyle, la conversion en sel hydrosoluble étant effectuée en introduisant directement dans le milieu réactionnel un alcoolate alcalin ou un sel alcalin d'un acide organique,
- on débarrasse le sel alcalin obtenu de ses impuretés résiduelles par extraction ou reflux à l'aide d'acétone.

5. Procédé selon l'une quelconque des revendications 1 à 4, dans lequel le sel soluble est formé par réaction avec l'acétate de sodium.

6. Vert d'Indocyanine contenant moins de 0,5 % d'impuretés résiduelles, exempt d'ions iodure, de traces d'amines cancérigènes et de solvants toxiques.

## Claims

1. A process for preparing substituted benz[e]-indoles cleared from residual impurities and substantially free from iodide ions and from traces of cancerigenous amines having the general formula (I) : 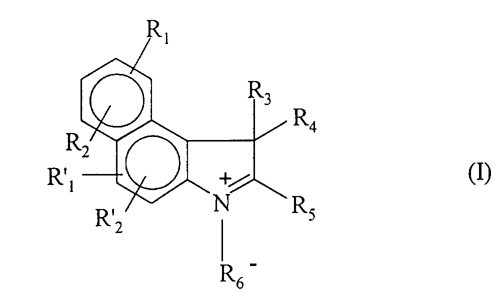 wherein
R₁, R₂, R'₁, R'₂, R₃ et R₄, which are the same or different one from the others represent hydrogen atoms, alkyl groups with C₁ to C₁₂, sulfoalkyl, cycloalkyl, or C₁-C₄ alkoxyl, aryl, aroxyl groups, or halogen atoms,
and R₆ represents a sulfo C₁-C₇ alkyl, preferably having from C₁ to C₄, and
R₅ represents a hydrogen atom, a C₁-C₁₂ alkyl group, a sulfoalkyl group, cycloalkyl group or a C₁-C₄ alkoxyl group, an aryl or aroxyl group, a halogen atom or a grouping of formula (I') 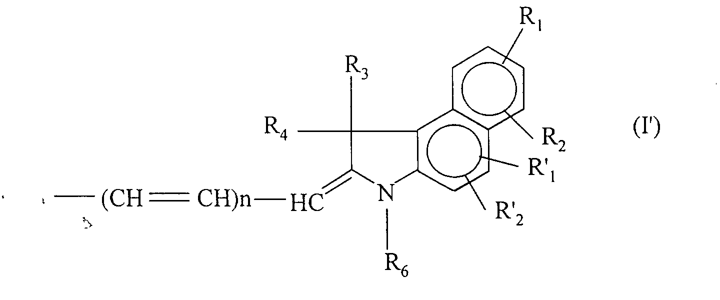 wherein n represents an integer from 1 to 7, with the proviso that when R₁, R₂, R'₁ et R'₂ represent a hydrogen and R₃ and R₄ a methyl, R₅ does not represent a methyl characterized by the fact that successively :
- an arylhydrazine of formula (III) : 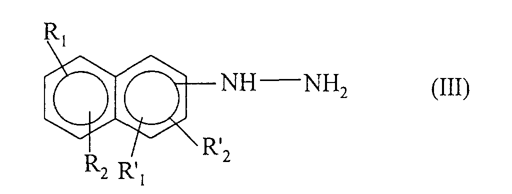 is prepared by reacting hydrazine with the corresponding arylhydroxyl
- a Fischer's indole synthesis is performed between the arylhydrazine of formula (III) and a ketone of formula (IV) : 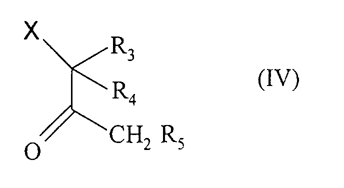 to provide the benz[e]indole of formula (V) : 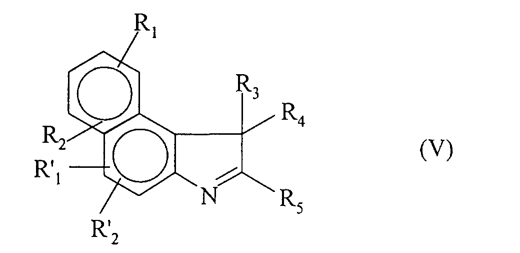 the sulfonic derivative of the benz[e]indole of formula (I) is prepared by reaction of the benz[e]indole of formula (V) with a C₁-C₇ sulfoalkyle, the meanings of R₁, R₂, R'₁, R'₂, R₃, R₄, and R₅ being those given regarding to formula (I), with the only difference that R₅ may not represent a grouping of formula (I'),
- the thus prepared benz[e]indole of formula (I) is converted into a soluble alkali metal salt thereof by reacting it with an alkali metal alcoolate or an alkali metal salt of an organic acid, being understood that when R₅ represents a grouping of formula (I'), the sulfonic derivative of benz[e]indole of formula (I) is at first reacted with the dianilide of glutaconic aldéhyde as the hydrochloride, then the thus obtained product is reacted with a second mole of sulfonic derivative of benz[e]indole of formula (I)
and the thus obtained alkali metal salt is freed from residual impurities by extraction or reflux by means of an apolar solvent, the boiling point of which is near of that of acetone.

2. A process according to claim 1, characterized by the fact that
in order to prepare the alkali metal salt of Indocyanine Green (Cardio Green) successively
- one prepares the benz[e]indole of formula (V) in which on one part R₁, R₂, R'₁ and R'₂ are hydrogen atoms and, on the other part R₃, R₄ et R₅ are methyl radicals, by reacting 2-naphtylhydrazine with isopropylmethylketone,
- one reacts the thus obtained benz[e]indole with 1,4-butane sultone,
- one condenses in acetic medium the resulting product of this reaction with the dianilide of glutaconic aldehyde as the hydrochloride, and
- one reacts in ethanolic medium the thus-formed product with a second mole of the benz[e]indole of formula (V) having on the nitrogen atom the same sulphobutylradical, the conversion of what into a water-soluble derivative being performed in directly introducing in the reaction mixture, an alkali metal alcoolate or an alkali metal salt of an organic acid,
- one clears the obtained product by extraction or reflux by means of acetone, of its impurities.

3. A process for preparing Indocyanine Green (Cardiogreen) containing less than 0,5 % residual impurities and substantially free of iodide ions and of traces of cancerigenous amines, having the general formula II : 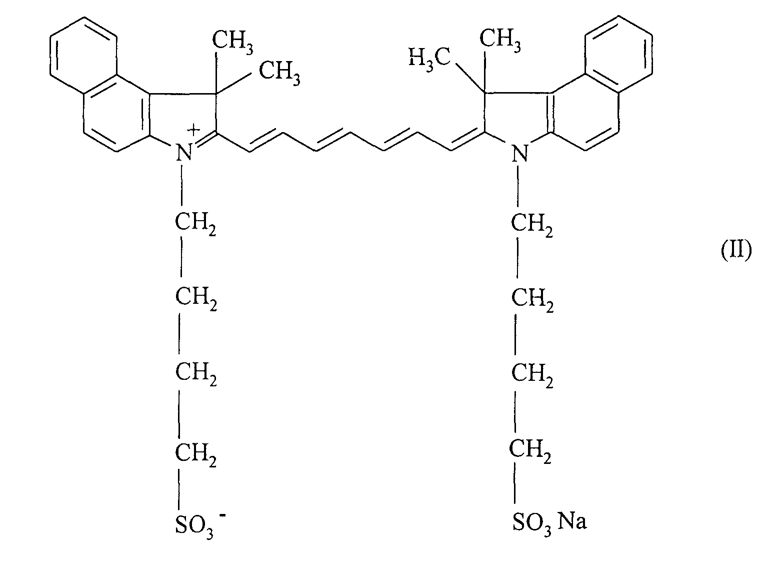 characterized by the fact that successively :
- one prepares an arylhydrazine of formula (III) : 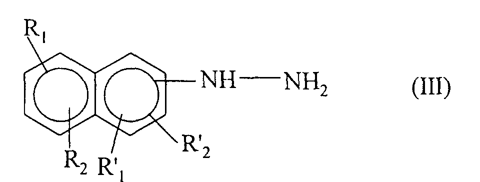 wherein R₁, R₂, R'₁ and R'₂ represent hydrogen atoms, by reacting hydrazine with the corresponding arylhydroxyl
- one performs a Fischer indole synthesis between the arylhydrazine of formula (III) and a ketone of formula (IV) : 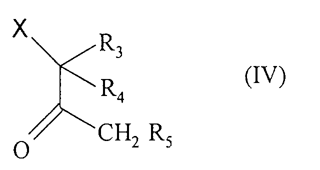 to provide a benz[e]indole of formula (V) : 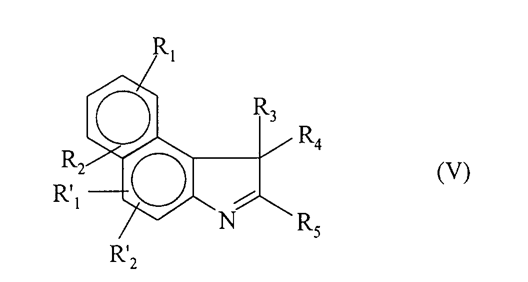 wherein R₃, R₄ and R₅ each represent a methyl group
- one forms the sulfonic derivative of benz[e]indole of formula (II) by reacting a benz[e]indole of formula (V) with a sulfo C₁-C₇ alkyle,
- one lets react the sulfonic derivative of benz[e]indole of formula (II) with the dianilide of glutaconic aldehyde as the hydrochloride, then the thus obtained product with a second molecule of the sulfonic derivative of the benz[e]indole of formula (II),
- one converts the thus prepared product into a soluble alkali metal salt thereof by reacting it with an alkali metal alcoolate or an alkali metal salt of an organic acid, and the thus obtained salt is purified by extraction or reflux with the help of acetone.

4. A process according to claim 3, characterized by the fact that for preparing the alkali metal salt of Indocyanine Green (Cardio Green), successively :
- one prepares the ben[z]indole of formula (V) wherein on one part R₁, R₂, R'₁ and R'₂ are each a hydrogen atom and on the other part R₃, R₄ et R₅ are methyl radicals, by reacting the 2-naphtylhydrazine with isopropylketone,
- one let the thus obtained benz[e]indole react with 1,4-butane-sulfone,
- one condenses in acetic medium the product of this reaction with the hydrochloride of the dianilide of glutaconic aldehyde and
- one let react in ethanolic medium the thus formed product with a second molecule of benz[e]indole of formula (V) bearing on the nitrogen atom the same sulfobutyl radical, the conversion thereof into a hydrosoluble salt being performed in directly introducing an alkali metal alcoolate or an alkali metal salt of an organic acid into the reaction mixture,
- one clears the obtained alki metal salt from its residual impurities by extraction or reflux by means of acetone.

5. A process according to any of claims 1 to 4 wherein the soluble salt is formed by reaction with sodium acetate.

6. Indocyanine Green (Cardio Green) containing less than 0,5 % residual impurities, free of iodide ions, traces of cancerigenous amines, and of toxic solvents.

## Patentansprüche

1. Verfahren zur Herstellung von substituierten Benz[e]indolen, befreit von ihren restlichen Verunreinigungen und im wesentlichen frei von lodidionen und Spuren von kanzerogenen Aminen, mit der allgemeinen Formel (I): 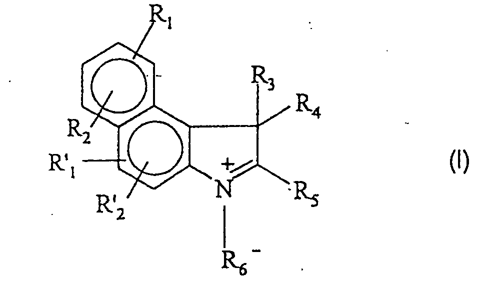 in der
R₁, R₂, R'₁, R'₂, R₃ und R₄, die identisch oder unterschiedlich sind, Wasserstoffatome, Alkylgruppen an C₁ bis C₁₂, Sulfoalkyl-,
Cycloalkyl-, Alkoxylgruppen an C₁ bis C₄, Aryl- bzw. Aroxylgruppen oder Halogenatome darstellen,
R₆ eine Sulfoalkylgruppe an C₁ bis C₇, vorzugsweise an C₁ bis C₄ darstellt, und
R₅ ein Wasserstoffatom, eine Alkylgruppe an C₁ bis C₁₂, eine Sulfoalkyl-, Cycloalkyl- oder Alkoxylgruppe an C₁-C₄, eine Aryl- oder Aroxylgruppe, ein Halogenatom oder eine Gruppe mit der Formel (I') 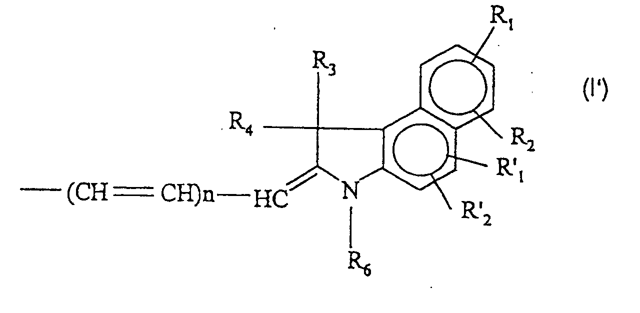 darstellt, in der n eine ganze Zahl von 1 bis 7 ist, unter der Bedingung, dass wenn R₁, R₂, R'₁ und R'₂ Wasserstoff und R₃, R₄ Methyl darstellen, R₅ nicht Methyl darstellt, dadurch gekennzeichnet, dass man nacheinander:
durch Reaktion von Hydrazin mit dem entsprechenden Arylhydroxyl ein Arylhydrazin mit der Formel (III) 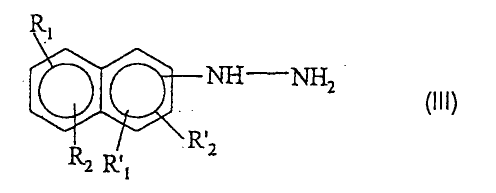 herstellt,
eine Fischer'sche Indolsynthese zwischen dem Arylhydrazin mit der Formel (III) und einem Keton mit der Formel (IV) 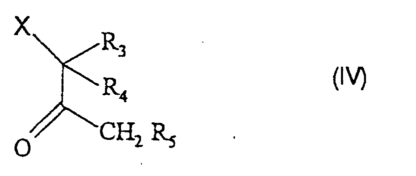
durchführt, was ein Benz[e]indol mit der Formel (V) ergibt: 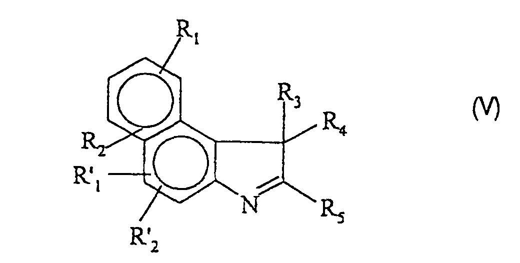 das Sulfonderivat des Benz[e]indols mit der Formel (I) durch Reaktion des Benz[e]indols mit der Formel (V) mit einem Sulfoalkyl an C₁-C₇ bildet, wobei die Bedeutung von R₁, R₂, R'₁, R'₂, R₃, R₄ und R₅ die gleiche ist wie die unter Bezugnahme auf die Formel (I) angegebene, mit der Ausnahme, dass R₅ keine Gruppe mit der Formel (I') darstellen kann,
das auf diese Weise hergestellte Benz[e]indol mit der Formel (I) durch Reaktion mit einem alkalischen Alkoholat oder einem Alkalisalz einer organischen Säure in ein lösliches Alkalisalz umwandelt, wobei als vereinbart gilt, dass wenn R₅ eine Gruppe mit der Formel (I') darstellt, man zuerst das Sulfonderivat von Benz[e]indol mit der Formel (I) mit Dianilidchlorhydrat von Glutaconaldehyd und dann das auf diese Weise hergestellte Produkt mit einem zweiten Molekül des Sulfonderivats von Benz[e]indol mit der Formel (I) reagieren lässt,
das auf diese Weise hergestellte Alkalisalz durch Extraktion oder Reflux mit Hilfe eines apolaren Lösemittels, dessen Siedepunkt demjenigen von Aceton nahe ist, von seinen restlichen Verunreinigungen befreit.

2. Verfahren gemäß Anspruch 1, dadurch gekennzeichnet, dass man zur Herstellung des Alkalisalzes von Indocyaningrün nacheinander: durch Reaktion von 2-Naphthylhydrazin mit Isopropylmethylketon das Benz[e]indol mit der Formel (V) herstellt, in der einerseits R₁, R₂, R'₁ und R'₂ Wasserstoffatome sind und andererseits R₃, R₄ und R₅ Methylradikale sind,
das auf diese Weise hergestellte Benz[e]indol mit 1,4-Butansulfon reagieren lässt,
das Resultat dieser Reaktion in saurem Milieu mit Dianilidchlorhydrat von Glutaconaldehyd kondensiert,
in einem Ethanolmilieu das auf diese Weise gebildete Produkt mit einem zweiten Benz[e]indol-Molekül mit der Formel (V) reagieren lässt, das am Stickstoffatom das gleiche Sulfobutylradikal aufweist, wobei die Umwandlung in ein wasserlösliches Salz ausgeführt wird, indem man direkt in das Reaktionsmilieu ein alkalisches Alkoholat oder ein Alkalisalz einer organischen Säure einführt, das hergestellte Produkt durch Extraktion oder Reflux mit Hilfe von Aceton reinigt.

3. Verfahren zur Herstellung von Indocyaningrün mit weniger als 0,5% restlichen Verunreinigungen, im wesentlichen frei von lodidionen und Spuren von kanzerogenen Aminen, mit der allgemeinen Formel (II): 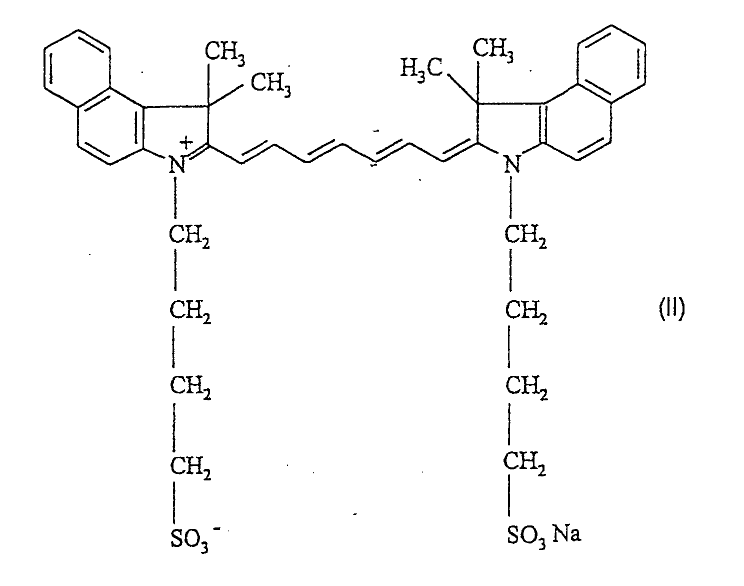 dadurch gekennzeichnet, dass man nacheinander:
- durch Reaktion von Hydrazin mit dem entsprechenden Arylhydroxyl ein Arylhydrazin mit der Formel (III) 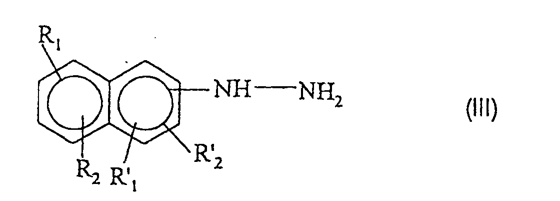 herstellt, in der R₁, R₂, R'₁ und R'₂ Wasserstoffatome darstellen, eine Fischer'sche Indolsynthese zwischen dem Arylhydrazin mit der Formel (III) und einem Keton mit der Formel (IV) 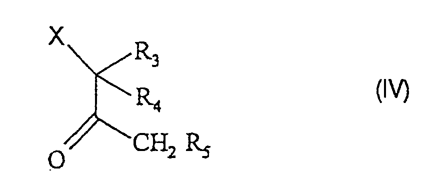 durchführt, was ein Benz[e]indol mit der Formel (V) 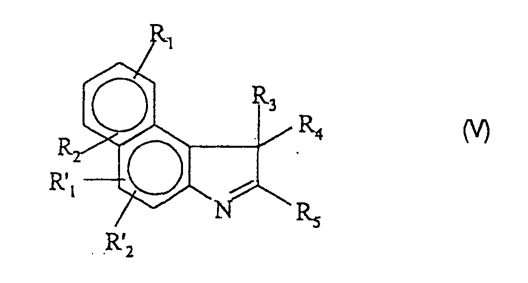 ergibt, in der R₃, R₄ und R₅ Methylgruppen darstellen, das Sulfonderivat des Benz[e]indols mit der Formel (II) durch Reaktion des Benz[e]indols mit der Formel (V) mit einem Sulfoalkyl an C₁-C₇ bildet,
das Sulfonderivat von Benz[e]indol mit der Formel (II) mit Dianilidchlorhydrat von Glutaconaldehyd und dann das auf diese Weise hergestellte Produkt mit einem zweiten Molekül des Sulfonderivats von Benz[e]indol mit der Formel (II) reagieren lässt, das auf diese Weise hergestellte Produkt durch Reaktion mit einem alkalischen Alkoholat oder einem alkalischen Salz einer organischen Säure in ein lösliches Alkalisalz umwandelt und das auf diese Weise hergestellte Salz durch Extraktion oder Reflux mit Hilfe von Aceton reinigt.

4. Verfahren nach Anspruch 3, dadurch gekennzeichnet, dass man zur Herstellung des Alkalisalzes von Indocyaningrün nacheinander:
- durch Reaktion von 2-Naphthylhydrazin mit Isopropylmethylketon das Benz[e]indol mit der Formel (V) herstellt, in der einerseits R₁, R₂, R'₁ und R'₂ Wasserstoffatome sind und andererseits R₃, R₄ und R₅ Methylradikale sind,
das auf diese Weise hergestellte Benz[e]indol mit 1,4-Butansulfon reagieren lässt,
das Resultat dieser Reaktion in saurem Milieu mit Dianilidchlorhydrat von Glutaconaldehyd kondensiert,
in einem Ethanolmilieu das auf diese Weise gebildete Produkt mit einem zweiten Benz[e]indol-Molekül mit der Formel (V) reagieren lässt, das am Stickstoffatom das gleiche Sulfobutylradikal aufweist, wobei die Umwandlung in ein wasserlösliches Salz ausgeführt wird, indem man direkt in das Reaktionsmilieu ein alkalisches Alkoholat oder ein Alkalisalz einer organischen Säure einführt,
das hergestellte Alkalisalz durch Extraktion oder Reflux mit Hilfe von Aceton von seinen Verunreinigungen befreit.

5. Verfahren nach einem der Ansprüche 1 bis 4, wobei das lösliche Salz durch Reaktion mit Natriumacetat gebildet wird.

6. Indocyaningrün mit weniger als 0,5% restlichen Verunreinigungen, frei von lodidionen, von Spuren kanzerogener Amine und von toxischen Lösemitteln.
